# EUROPEAN PATENT APPLICATION

(11) **EP 2 364 647 A1**
(43) Date of publication of application: **14.09.2011**
(21) Application number: 10155985.4
(22) Date of filing: 09.03.2010
(51) Int. Cl.: A61B 6/00, A61B 5/05

(54) **Automatic electromagnetic apparatus for detection and diagnosys of anomalies in biological tissues**

(71) Applicant: Centro Studi e Ricerche Sant' Angela Srl, 20157 Milano (IT)
(72) Inventor: Bellorofonte, Carlo, 20157 Milano (IT)
(74) Representative: Serravalle, Marco

(57) **Abstract**

The present invention concerns an apparatus for the determination of anomalies in biological tissues, which apparatus comprises the following components: a radio transmitter irradiating a radio frequency; a radio receiver capable of receiving the frequency irradiated by the transmitter; a data processing unit which processes the signal received by the radio receiver.

In another embodiment the invention is directed to a method for automatic diagnosys of cellular anomalies, which method comprises the following steps: positioning the person which has to undergo examination in the right position; positioning the apparatus of claims 1-8 in the starting position; starting the automatic scanning; printing the results of the scanning.

## Description

The present invention relates to an apparatus for the detection of cellular anomalies. In another embodiment of the invention, it is described a method for the automatic diagnosis of cellular anomalies.

### State of the Art

WO 01/07909 (Vedruccio) describes the use an apparatus making use of a coherent tranceiver probe which generates radiofrequencies at three different wavelengths (436, 872 and 1308 MHz) and a spectrum analyser. The probe is manually swept along the body and the decrease or disappearance of one of the three peaks is considered as an indication of a type of alteration. Because of this, the three different wavelengths are considered essential.

This patent application has been implementated in a commercially available apparatus usually called Trimprobe. This apparatus has been used by several doctors but is considered scarcely reliable since the results are strongly dependent on the operator. In fact, when using the apparatus, the doctor moves the probe along the body of the patient; if the probe is rotated, we can assist to sudden changes in the intensity of the three peaks. Furthermore, the relative position of the patient, the probe and the receiver strongly infleunces the results. Another problem is the disturb which can be present when, for example, a person moves around the apparatus: one of the three signals can significantly lower simply because of the movement of the person around the machine. It is therefore difficult for doctors to rely on this apparatus for the diagnosys of an altered state of human tissues.

### Description of the invention

The present invention intends to overcome the above described problems by providing an apparatus for the detection of cellular anomalies which is reliable, and whose results are reproducible and independent on the operator making use of the apparatus.

The invention relates to an apparatus for the determination of anomalies in living tissues, which apparatus comprises the following parts: a radio transmitter irradiating a frequency of from 100 MHz to 1000 MHz; a radio receiver capable of receiving the frequency irradiated by the transmitter; and a data processing unit which processes the signal received by the radio receiver, wherein the radio transmitter and the radio receiver are interconnected and synchronised.

In a preferred embodiment the apparatus according to the invention comprises a software for the automatic scanning of the human body. In this way, the apparatus according to the invention does not need to be operated by a doctor and can be used by a paramedical. Once the person who needs to undergo the examination is placed in the right position, for example he or she lies on a bed connected to the apparatus, the apparatus can perform the scanning in an automatic way. In a preferred ambodiment, at the end of the scanning, the apparatus provides a paper diagram wherein possible areas of alteration are evidenced in a x-y plot, so that the doctor, when receiving the results, will be able to understand which organ(s) are possibly interested by the anomaly.

In this preferred embodiment, the apparatus will also comprise an electric engine which allows the automatic movement of the transmitter and of the receiver in the x, y and possibly also z axys.

An important aspect of the invention is that receiver and transmitter are interconnected and synchronised. It means that when moving the receiver along the body of the person, the transmitter also moves correspondently, so that their relative position remains unchanged. In a preferred embodiment, the person undergoing examinantion lies on a bed and either the transmitter or the receiver are placed above the body. In a more preferred embodiment, the transmitter is placed above the body and the receiver below it.

In a preferred embodiment, the transmitter is placed in close contact with the body of the patient. For this purpose, the transmitter can be mounted on a ball-and-socket joint. In this way, the transmitter can stay in contact with the body and adapt to its shape.

In another preferred embodiment, the transmitter is placed at a few centimeters from the body of the patient, for example at a distance comprised between 2 and 25 cm, more preferably between 3 and 20 cm.

During the scanning, the distance between transmitter and receiver preferably remains unchanged; however, they need not stay parallel during the entire scanning. In fact, if the apparatus comprises a ball-and-socket joint, the transmitter can deviate from the parallel position in relation to the receiver without that the receiver looses the signal of the transmitter.

In a preferred embodiment, synchronised transmitter and receiver can also move along the z axys, i.e. in the direction perpendicular to the body. More preferably, the tranmitter and receiver can move independently one from the other before starting the scanning. In this way, the machine can adapt the distance between transmitter and receiver and optimise it as a

Preferably, the distance between transmitter and receiver is comprised between 50 cm and 150 cm, more preferably between 60 cm and 120 cm.

The type of antenna used as transmitter and receiver is not particularly limited, since any type of antenna can be used as long as they can communicate each other. However, in view of possible radio signals present in the air, it is preferred that the antenna are directional antenna, so that the signal emitted by the transmitter is focussed in the direction of the receiver, minimizing any interference with external radiofrequencies.

The power of the radiofrequency emitted by the transmitter can vary in a broad range, however, it is preferable to limit the power of the radiofrequency in the range from 1 to 200 mW, preferably from 5 to 100 mW, more preferably from 10 to 50 mW.

The frequency used by the apparatus according to the invention can vary in a broad range. This finding is in sharp contrast with the teaching of the prior art which focusses on the importance of the frequency of 463 MHz and multiples. These are the frequencies tought by Vedruccio and used by Trimprobe. However, it has been found that it is possible to operate the apparatus of the invention at different frequencies, such as 800 MHz. In a preferred embodiment, the apparatus uses a frequency comprised between 100 and 1000 MHZ, more preferably between 200 and 1000 MHz, most preferably between 400 and 1000 MHz.

Another embodiment of the invention is directed to a method for automatic diagnosys of cellular anomalies, which method comprises the following steps: positioning the person which has to undergo examination in the right position, for example lying on a bed; positioning the apparatus in the starting position; starting the automatic scanning; printing the results of the scanning.

This method can be performed with the assistence of a paramedical, since the apparatus simply requires the positioning in the starting position and a person to start scanning. Once the results are printed, they are read by a doctor.

## Claims

1. Apparatus for the determination of anomalies in biological tissues, which apparatus comprises the following components:
a. a radio transmitter irradiating a radio frequency;
b. a radio receiver capable of receiving the frequency irradiated by the transmitter;
c. a data processing unit which processes the signal received by the radio receiver;
**characterized in that** the radio transmitter and the radio receiver are interconnected and synchronised.

2. Apparatus according to claim 1, wherein the radiofrequency is comprised between 100 and 1000 MHz.

3. Apparatus according to claims 1-2, further comprising a software for the automatic scanning of the human body.

4. Apparatus according to claims 1-3 further comprising an electric engine to move the transmitter and the receiver along the body of the patient.

5. Apparatus according to claims 1-4 further comprising a bed on which the patient lyes during the scanning.

6. Apparatus according to claims 1-5 wherein the transmitter is in close contact with the human body.

7. Apparatus according to claim 1 wherein the distance between the transmitter and the body is comprised between 2 and 25 cm.

8. Apparatus according to claims 1-7 wherein the transmitter emits a radiofrequency with a power in the range of from 1 mW to 200 mW.

9. Method for automatic diagnosys of cellular anomalies, which method comprises the following steps: positioning the person which has to undergo examination in the right position; positioning the apparatus of claims 1-8 in the starting position; starting the automatic scanning; printing the results of the scanning.
